(19) Europäisches Patentamt
European Patent Office
Office européen des brevets

(11) Veröffentlichungsnummer: **0 137 390 B1**

(12) # EUROPÄISCHE PATENTSCHRIFT

(45) Veröffentlichungstag der Patentschrift: **16.08.90**

(51) Int. Cl.[5]: **C 07 D 471/04,** A 61 K 31/44

(21) Anmeldenummer: **84111337.6**

(22) Anmeldetag: **22.09.84**

(54) **Substituierte beta-Carboline, Verfahren zu ihrer Herstellung und ihre Verwendung als Arzneimittel.**

(30) Priorität: **27.09.83 DE 3335323**

(43) Veröffentlichungstag der Anmeldung:
**17.04.85 Patentblatt 85/16**

(45) Bekanntmachung des Hinweises auf die Patenterteilung:
**16.08.90 Patentblatt 90/33**

(84) Benannte Vertragsstaaten:
**AT BE CH DE FR GB IT LI LU NL SE**

(56) Entgegenhaltungen:
**EP-A-0 030 254 EP-A-0 110 813**
**EP-A-0 054 507 DE-A-1 470 396**

**PATENT ABSTRACTS OF JAPAN, unexamined applications, Sektion C, Band 6, Nr 63, 22. April 1982, THE PATENT OFFICE JAPANESE GOVERNMENT, Seite 138 C 99**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Huth, Andreas, Dr.**
**Kyllmannstrasse 15**
**D-1000 Berlin 39 (DE)**
Erfinder: **Schmiechen, Ralph, Dr.**
**Bayernring 27**
**D-1000 Berlin 42 (DE)**
Erfinder: **Rahtz, Dieter, Dr.**
**Krottnaurerstrasse 24a**
**D-1000 Berlin 38 (DE)**
Erfinder: **Seidelmann, Dieter, Dr.**
**Stierstrasse 14**
**D-1000 Berlin 41 (DE)**
Erfinder: **Breastrup, Claus Thyco, Dr.**
**Frederiksborgvej 78**
**DK-4000 Roskilde (DK)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 137 390 B1

**Beschreibung**

Die Erfindung betrifft neue substituierte α-Carboline, Verfahren zu ihrer Herstellung und ihre Verwendung als Arzneimittel gemäß den Ansprüchen.

Die erfindungsgemäßen Verbindungen besitzen wertvolle pharmakologische Eigenschaften. Sie beeinflussen insbesondere das Zentralnervensystem und eignen sich somit als Psychopharmaka.

3-Alkoxycarbonyl-β-carboline, die im A-Ring mit einem Kohlenwasserstoffrest substituiert sind, werden in der EP—A—54507 und in der JP—A—574987 beschrieben.

Die neuen β-Carboline besitzen die allgemeine Formel I

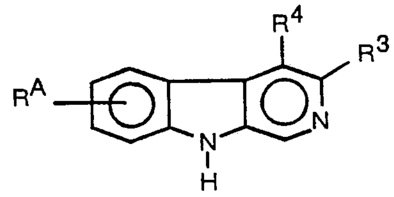

(I),

wobei

$R^3$ einen Oxadiazolylrest der Formel

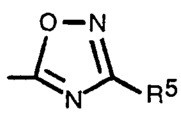

mit $R^5$ als niederem Alkyl mit bis zu 3 C-Atomen oder einen Ester

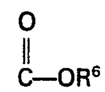

mit $R^6$ als Wasserstoff oder niederem Alkyl mit bis zu 3 C-Atomen,

$R^4$ Wasserstoff, niederes Alkyl mit bis zu 3-C-Atomen oder $CH_2OR^9$ mit $R^9$ als niederem Alkyl mit bis zu 3 C-Atomen,

$R^A$ Phenyl, einen verzweigten ode unverzweigten Alkylrest mit 2—10 Kohlenstoffatomen, einen Phenethyl- oder Cyclohexylvinylrest oder einen Cycloalkyl-, verzweigten oder unverzweigten Alkenyl- oder Cycloalkenylrest mit 2—10 Kohlenstoffatomen, wobei im Cycloalkylrest eine $CH_2$-Gruppe durch Carbonyl oder Sauerstoff ersetzt sein kann, darstellen, wobei, falls $R^A$ einen Phenethylrest oder einen $C_{2-10}$-Alkylrest bedeutet, $R^3$ eine Oxadiazolylrest ist.

Die neuen β-Carboline der allgemeinen Formel I sind in 3-Stellung mit einem substituierten [1.2.4]-Oxadiazol-5-yl-rest oder mit einem Alkyloxycarbonylrest substituiert, wobei der Substituent am Oxadiazolylrest und an der Oxycarbonylgruppe in beiden Fällen niederes Alkyl mit bis zu 3 C-Atomen darstellt. Beispielsweise seien genannt Methyl, Ethyl, Propyl und iso-Propyl.

In 4-Stellung weisen die neuen β-Carboline entweder Wasserstoff, niederes Alkyl wie Methyl oder Ethyl oder eine Niederalkoxymethylgruppe auf.

Der Substituent $R^A$ stellt Phenyl, einen verzweigten oder unverzweigten Alkylrest mit 2—10 Kohlenstoffatomen, einen Phenethyl- oder Cyclohexylvinylrest oder einen Cycloalkyl-, verzweigten oder unverzweigten Alkenyloder Cycloalkenylrest mit 2—10 Kohlenstoffatomen dar. Genannt seien verzweigte und unverzweigte Alkylreste wie beispielsweise 2-Pentyl, n-Butyl, tert.-Butyl, n-Hexyl, 3-Methylbutyl, 2,3-Dimethylbutyl.

Bedeutet $R^A$ einen Cycloalkylrest, so kommen die folgenden Reste beispielsweise in Betracht: Cyclopentyl, Cyclohexyl, Cycloheptyl und Cyclooctyl.

Im Cycloalkylrest kann eine $CH_2$-Gruppe gegebenenfalls durch eine Carbonylgruppe oder durch ein Sauerstoffatom ersetzt sein.

Als Alkenylreste $R^A$ kommen beispielsweise 1,3-Butadienyl, 2,3,-Dimethyl-1,3-Butadienyl, 3-Methyl-1,3-butadienyl in Betracht und als Cycloalkenylreste seien beispielsweise genannt 1-Cyclohexen-4-yl, 4-Cyclohepten-1-yl, 1-Cycloocten-1-yl, 2-Cyclohexen-4-yl, 3-Cyclohepten-1-yl.

Der Substituent $R^A$ kann sich in 5- oder 6-Stellung befinden, wobei die 6-Stellung bevorzugt ist.

Es ist bekannt, daß bestimmte Stellen im zentralen Nervensystem von Vertebraten eine hohe spezifische Affinität für die Bindung von 1.4- und 1.5-Benzodiazepinen aufweisen (Squires, R. F. und Braestrup, C., Nature (London) 266(1977)734). Die Stellen werden Benzodiazepin-Rezeptoren genannt.

Es wurde gefunden, daß die erfindungsgemäßen substituierten β-Carboline, obwohl sie sich in ihrer chemischen Struktur von den Benzodiazepinen stark unterscheiden, überraschenderweise eine strake Affinität und Spezifität für die Bindung an die Benzodiazepin-Rezeptoren zeigen, indem sie radioaktivmarkiertes Flunitrazepam von diesen Benzodiazepin-Rezeptoren verdrängen.

Die Verdrängungsaktivität der erfindungsgemäßen Verbindungen ist in der nachfolgenden Tabelle als $IC_{50}$- und $ED_{50}$-Wert angegeben. Der $IC_{50}$-Wert gibt die Konzentration an, die eine 50%ige Verdrängung der spezifischen Bindung von $^3H$-Flunitrazepam (1,0 nM, 0°C) in Proben mit einem Gesamtvolumen von 0,55 ml einer Suspension von Hirnmembran, z.B. von Ratten, bewirkt.

Die Verdrängungsaktivität wird im in vitro Test wie folgt bestimmt: 0,5 ml einer Suspension von unbehandeltem Ratten-Haupthirn in 25 mM $KH_2PO_4$, pH = 7,1 (5—10 mg Gewebe/Probe) wird für 40—60 Minuten bei 0°C zusammen mit $^3H$-Diazepam (spezifische Aktivität 14,4 Ci/mMol, 1,9 nM) oder $^3H$-Flunitrazepam (spezifische Aktivität 87 Ci/mMol, 1,0 nM) inkubiert. Nach Inkubation wird die Suspension durch eine Glasfritte filtriert, der Rückstand 2mal mit kalter Pufferlösung gewaschen und die Radioaktivität am Scintillationszähler gemessen.

Der Versuch wird dann wiederholt, jedoch so, daß vor der Zugabe des radioaktiv-markierten Benzodiazepins eine bestimmte Menge oder eine überschüssige Menge der Verbindung, deren Verdrängungsaktivität zu bestimmen ist, zugesetzt wird. Auf Grundlage der erhaltenen Werte wird der $IC_{50}$-Wert berechnet.

Der $ED_{50}$-Wert stellt die Dosis eineer Versuchssubstanz dar, die eine Reduktion der spezifischen Bindung des Flunitrazepams an dem Benzodiazepin-Rezeptor in einem lebenden Gehirn auf 50% des Kontrollwertes bewirkt.

Der in-vivo Test wird wie folgt ausgeführt:

Gruppen von Mäusen wird die Versuchssubstanz bei unterschiedlichen Dosen und normalerweise subcutan injiziert. Nach 15 Minuten wird dann den Mäusen das $^3H$-Flunitrazepam intravenös verabreicht. Nach weiteren 20 Minuten werden die Mäuse getötet, ihre Vorderhirnhäute entfernt und die Radioaktivität der Vorderhirnhäute durch Scintillationszählung gemessen. Der $ED_{50}$-Wert wird mit Hilfe der Dosis/Wirkungs-Kurven bestimmt.

Die Ergebnisse der pharmakologischen Teste sind in der nachfolgenden Tabelle zusammengestellt.

TABELLE

Verdrängungsaktivität von substituierten β-Carbolinderivaten der Formel I

| Substituent | | $R^A$ | | | |
|---|---|---|---|---|---|
| $R^3$ | $R^4$ | $R^5$ | $R^6$ | $IC_{50}$ ng/ml (in vitro) | $ED_{50}$ mg/ml (in vivo) |
| $CO_2Me$ | H | H | H *) | 1,9 | 22 |
| $CO_2Et$ | Me | H | 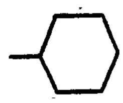 | 12 | 4,7 |
| $CO_2Et$ | Me | H | 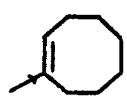 | 19 | 11 |
| $CO_2Et$ | $CH_2OCH_3$ | H | 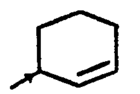 + 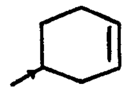 1 : 4 | 0,7 | 0,5 |
| 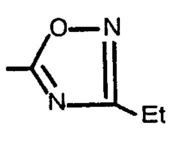 | $CH_2OCH_3$ | H | 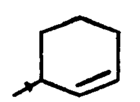 + 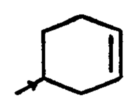 1 : 4 | 1,5 | 1,9 |
| $CO_2Et$ | $CH_2OCH_3$ | $CH_2—C_6H_5$ | H | 0,5 | 3,5 |
| $CO_2Et$ | Me | H | 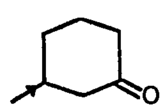 | 0,6 | 0,9 |

*) Nature *294*(1981)472

Die erfindungsgemäßen Verbindungen erscheinen aufgrund ihrer biologischen Wirksamkeit als Psychopharmaka für die Humanmedizin geeignet. Sie können hierzu psychopharmazeutischen Präparationen, zum Beispiel für die orale und parenterale Anwendung, formuliert angewendet werden.

Als Formulierungshilfsstoffe sind physiologisch verträgliche organische und anorganische Trägersubstanzen geeignet, die gegenüber den erfindungsgemäßen Verbindungen inert sind.

Als Trägersubstanzen seien beispielsweise genannt Wasser, Salzlösungen, Alkohole, Polyäthylenglykole, polyhydroxyethoxyliertes Rizinusöl, Gelatine, Lactose, Amylose, Magnesiumstearat, Talkum, Kieselsaüre, Fettsäuremono- und diglyceride, Pentaerythritolfettsäureester, Hydroxymethylcellulose und Polyvinylpyrrolidon.

Die pharmazeutischen Präparationen können sterilisiert und/oder mit Hilfsstoffen, wie Schmiermittel, Konservierungsstoffen, Stabilisatoren, Netzmittel, Emulgatoren, Puffermittel und Farbstoffen, versetzt werden.

Für die parenterale Anwendung sind insbesondere Innjektionslösungen oder Suspensionen, insbesondere wäßrige Lösungen der aktiven Verbindungen in polyhydroxyethoxyliertem Rizinusöl, geeignet.

Für die orale Anwendung sind insbesondere Tabletten, Dragees oder Kapseln mit Talkum und/oder einem Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls en Süßstoff beigefügt wird.

Die erfindungsgemäßen Verbindungen werden in einer Dosiseinheit von 0,05 bis 10 mg aktiver Substanz in einem physiologisch verträglichen Träger eingebracht.

Die erfindungsgemäßen Verbindungen werden in einer Dosis von 0,1 bis 300 mg/Tag, vorzugsweise 1—30 mg/Tag, angewendet.

Die Herstellung der erfindungsgemäßen Verbindungen der allgemeinen Formel I erfolgt nach an sich bekannten Methoden, dadurch daß man

A. ein halogeniertes β-Carbolinderivat der allgemeinen Formel II

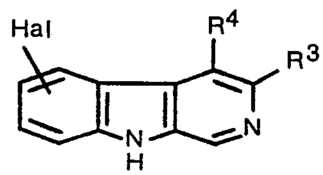

(II),

worin Hal für Brom oder Jod steht und $R^3$ und $R^4$ die oben angegebene Bedeutung haben, mit einem Alkenyl- oder Cycloalkenylrest mit 2—10 C-Atomen, in einem aprotischen polaren Lösungsmittel in Gegenwart eines Schwermetallsalzes und einer Base unter Druck umsetzt, und anschließend gegebenenfalls die im Substituenten $R^A$ vorhandene isolierte oder konjugierte Doppelbindung in Gegenwart von Raney-Nickel oder einem Edelmetallkatalysator auf einem Trägermaterial in einem protischen Lösungsmittel hydriert oder anschließend mit elementarem Schwefel in Dimethylsulfoxid oder mit Palladium in Xylol und/oder Mesitylen dehydriert und anschließend gegebenenfalls eine Estergruppe in 3-Stellung alkalisch hydrolysiert und gegebenenfalls anschließend die so erhaltene freie Säure der Formel III

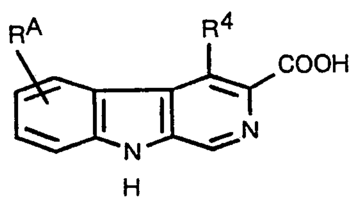

(III),

worin $R^4$ und $R^A$ die oben angegebene Bedeutung haben, mit einem Amidoxim der allgemeinen Formel $R^5—C(=NOH)NH_2$, worin $R^5$ die oben angegebene Bedeutung hat, in einem inerten Lösungsmittel bei Raumtemperatur bis zur Siedetemperatur des Reaktionsgemisches umsetzt, oder

B. ein β-Carbolinderivat der Formel IV

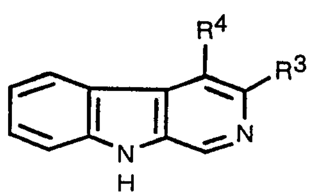

(IV),

wobei $R^3$ und $R^4$ die oben angegebene Bedeutung haben, mit einem Alkylhalogenid oder einem Alken mit 2 bis 10 C-Atomen und Halogenid in der Bedeutung von Chlor oder Brom in Gegenwart von Aluminiumtrichlorid bei Raumtemperatur alkyliert oder

C. ein substituiertes β-Carbolinderivat der Formel V

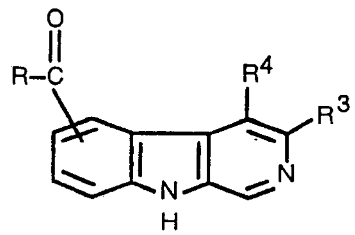

(V),

wobei R Wasserstoff oder einen Kohlenwasserstoffrest mit bis zu 9 C-Atomen darstellt und $R^3$ und $R^4$ die oben angegebene Bedeutung haben, in Gegenwart von Palladium in feinverteilter Form in einem aliphatischen Alkohol und Eisessig bei Temperaturen zwischen 20 und 100°C und Drücken von 5—20 bar hydriert oder

D. ein substituiertes Indol der Formel VI

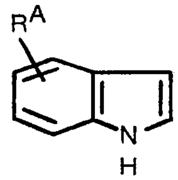

(VI),

worin $R^A$ die oben angegebene Bedeutung hat, mit einem Azadien der Formel VII

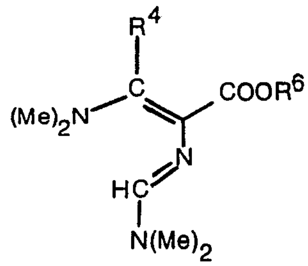

(VII),

wobei $R^4$ die oben angegebene Bedeutung hat und $R^6$ einen niederen Alkylrest mit bis zu 3 C-Atomen darstellt, in Gegenwart einer Säure bei Temperaturen von 50—200°C umsetzt oder

E. ein substituiertes Indol der Formel VI mit einem 4-Alkoxy-3-hydroxy-2-nitro-buttersäurealkylester der Formel VIII

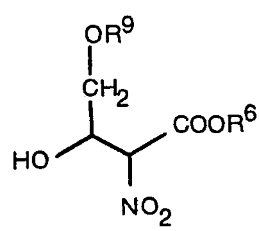

(VIII),

worin $R^6$ und $R^9$ die oben angegebene Bedeutung haben, in einem inerten Lösungsmittel bei Rückflußtemperatur in Gegenwart einer aliphatischen Carbonsäure erhitzt, anschießend den so erhaltenen 3-Indol-3-yl)-2-nitro-5-oxa-hexanalkylester der Formel IX

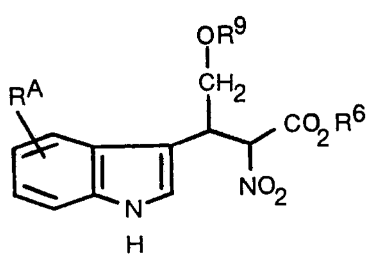

(IX),

worin $R^6$ und $R^9$ die oben angegebene Bedeutung haben, in Gegenwart von Raney-Nickel bei Raumtemperatur und unter Normaldruck zur entsprechenden 2-Amin -Verbindung hydriert, mit Glyoxylsäure bei einem pH von 3—5 bei Raumtemperatur umsetzt, wobei ein $R^A$-substituierter 4-Alkoxymethyl-1.2.3.4-tetrahydro-β-carbolin-1-carbonsäure-3-carbonsäurealkylester der Formel X

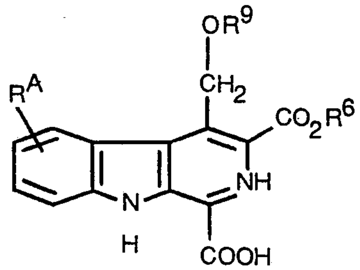

(X),

worin $R^6$ und $R^9$ und $R^A$ die oben angegebene Bedeutung haben, entsteht, der anschließend in einem hochsiedenden inerten Lösungsmittel durch Erhitzen auf Rückflußtemperatur decarboxyliert und anschließend dehydriert.

Die Herstellung der erfindungsgemäßen Verbindungen der allgemeinen Formel I erfolgt nach an sich bekannten Methoden.

Zurt Herstellung von Verbindungenen der Formel I, in denen $R^A$ einen Alkenyl-oder Cycloalkenylrest mit 2 bis 10 C-Atomen darstellt, wird ein im A-Ring des β-Carbolinmoleküls mit Halogen, wie Brom oder Jod substituiertes β-Carbolin der Formel II mit einem Alkenyl-oder Cycloalkenylrest, wie er oben als Rest definiert wurde, in einem aprotischen polaren Lösungsmittel in Gegenwart eines Schwermetallsalzes und einer Base unter Druck umgesetzt.

Als aprotische polare Lösungsmittel seien beispielsweise genannt Dimethylformamid, Methylpyrrolidon, Hexamethylphosphortriamid, Dimethylacetamid, Acetonitril und Trimethylenglycoldimethylether.

Als Schwermetallsalze sind insbesondere die gebraüchlichen Chloride, Sulfate und Acetate von Ruthenium, Rhodium, Palladium und Platin zusammen mit organischen Phosphorverbindungen wie Triphenylphosphin und Tri-o-tolylphosphin geeignet.

Als Basen sind an sich alle organischen und anorganischen Basen geeignet. Gut bewährt hat sich indesen beispielsweise tert.-Butylamin und Natriumhydrogencarbonat.

Die Reaktion wird zweckmäßigerweise in einer Inertatmosphäre wie Stickstoff oder Edelgas bei Temperaturen von 70—150°C unter Druck in einem Bereich von 1—5 atü (1,96—6,08 Bar) ausgeführt.

Die sich gegebenenfalls anschließende Hydrierung, bei der isolierte und konjugierte Doppelbindungen und Dreifachbindungen, jedoch keine aromatische Doppelbindungen reagieren, liefert Verbindungen der Formel I, bei denen der Substituent $R^A$ gesättigt ist. Hierzu wird das Ausgangsmaterial in einem protischen Lösungsmittel, z.B in einem aliphatischen Alkohol wie Methanol oder Ethanol in Gegenwart von Raney-Nickel oder einem Edelmetallkatalysator auf einem geeigneten Trägermaterial wie Palladium auf Kohle hydriert. Zweckmäßigerweise wird die Hydrierung unter Druck im Bereich von 1 bis 20 bar, vorzugsweise bei 5—10 bar ausgeführt.

Die sich gegebenenfalls anschließende Dehydrierung von Substituenten $R^A$, die eine isolierte oder konjugierte Doppelbindung aufweisen, liefert Verbindungen, bei denen $R^A$ einen aromatischen Rest, wie die Phenylgruppe, darstellt. Hierzu wird das Ausgangsmaterial entweder in Dimethylsulfoxid mit elementarem Schwefel oder in Xylol oder Mesitylen oder einem Gemisch davon mit Palladium auf Kohle in der Wärme bei 150—200°C behandelt.

Die sich gegebenenfals anschließende Verseifung einer Estergruppe in 3-Stellung erfolgt zweckmäßigerweise alkalisch, wobei der Ester mit verdünnter wässriger Alkalilauge, wie Kalium- oder Natriumhydroxid, in einem protischen Lösungsmittel, wie z.B. Methanol, Ethanol oder Ethylenglykol, auf Temperaturen bis zur Rückflußtemperatur des Reaktionsgemisches erhitzt wird.

Die so in der Vorstufe erhaltene freie β-Carbolin-3-carbonsäure der Formel III dient zur Herstellung von Verbindungen der Formel I, in der $R^3$ den 5-Oxadiazolylrest darstellt. Hierzu wird die β-Carbolin-3-carbonsäure mit einem Amidoxim der Formel $R^5—C(=NOH)NH_2$, wobei $R^5$ einen Niederalkylrest darstellt, in einem Lösungsmittel, das über 100°C siedet und gegenüber den Reaktanten inert ist, bei der Rückflußtemperatur des Reaktionsgemisches zur Kondensation gebracht. Geeignete Lösungsmittel für de Kondensationsreaktion sind beispielsweise Toluol und Dimethylformamid. Zweckmäßigerweise wird die freie β-Carbolin-3-carbonsäure vor de Kondensationsreaktion in geeigneter Weise aktiviert. Hierzu kann die freie Säure in das gemischte Anhydrid, in den aktivierten Ester oder in das Chlorid überführt werden. Gut bewährt hat sich eine Aktivierung mit Imidazol/Thionylchlorid in einem aprotischen Lösungsmittel wie Dioxan, Tetrahydrofuran, Dimethylformamid oder N-Methylpyrrolidon bei Temperaturen zwischen 0 und 50°C, vorzugsweise Raumtemperatur.

Ein anderer Weg zur Herstellung von Verbindungen der Formel I ist die Alkylierung nach Art einer Friedel-Craftsschen Reaktion. Hierzu läßt man das entsprechende Alkylchlorid auf das im A-Ring unsubstituierte β-Carbolin in Gegenwart von Aluminiumtrichlorid bei Raumtemperatur einwirken, wobei gleichzeitig neben monoalkylierten auch dialkylierte Endprodukte, z.B. in 6- und 8- Stellung, entstehen, die sich aber leight, z.B. durch Umkristallisation, abtrennen lassen.

Eine andere Synthesemöglichkeit zur Herstellung von Verbindungen der Formel I, in denen $R^A$ für einen Alkylrest steht, ist die Reduktion einer Keto- oder Aldehydfunktion am A-Ring des β-Carbolinmoleküls. Hierzu wird ein β-Carbolinderivat der Formel V in Gegenwart von Palladium in feinverteilter Form, z.B. als Palladium-Mohr, in einem aliphatischen Alkohol wie Methanol oder Ethanol und einer aliphatischen Carbonsäure wie Essigsäure unter Druck im Bereich von 4—20 bar bei Temperaturen zwischen Raumtemperatur und 100°C hydriert.

Eine weitere Synthesemöglichkeit zur Herstellung von Verbindungen der Formel I ist die Umsetzung eines $R^A$-substituierten Indols der Formel VI mit einem Azadien der Formel VII.

Die Umsetzung des Indolderivats mit dem Azadien erfolgt in Gegenwart von Säuren bei Temperaturen zwischen 50 und 200°C, vorzugsweise bei 75 bis 150°C. Dioe Umsetzung wird beispielsweise so ausgeführt, daß das Indolderivat der Formel VI und das Azabutadien der Formel VII in einer aliphatischen Carbonsäure wie Ameisensäure, Essigsäure, Propionsäure oder Trifluoressigsäure, oder in einem anorganischen Milieu, wie in Phosphorsäure, Polyphosphorsäure oder Phosphoroxychlorid usw. erhitzt werden. Es können auch inerte organische Lösungsmittel, wie zum Beispiel Toluol, Ethylacetat, Dioxan, Dimethoxyethan, Acetonitril u.a. zugesetzt werdfen.

Die Umsetzung kann aber auch in Gegenwart von katalytischen Mengen einer Mineralsäure wie Schwefelsäure, Salzsäure, Perchlorsäure etc. in einem der zuvor genannten inerten Lösungsmittel ausgeführt werden.

Die Umsetzung ist nach 3—10 Stunden abgeschlossen. Ihr Verlauf kann zum Beispiel durch Dünnschichtchromatographie verfolgt werden.

Eine andere Synthesemöglichkeit zur Herstellung von Verbindungen der Formel I aus $R^A$-substituierten Indol-derivaten der Formel VI ist die Umsetzung mit einem 4-Alkoxy-3-hydroxy-2-nitro-buttersäurealkylester der Formel VIII. Hierzu werden die Reaktanten in einem inerten Lösungsmittel wie Benzol, Toluol oder Xylol in Gegenwart einer aliphatischen Carbonsäure wie Essigsäure auf Rückflußtemperatur erhitzt. Zweckmäßigerweise wird diese Reaktion unter Schutzgasatmosphäre ausgeführt.

Das so erhaltene Reaktionsprodukt 3-(4-Alkoxyindol-3-yl)-2-nitro-5-oxa-hexanalkylester der Formel IX wird bei Raumtemperatur und Normaldruck mit Raney-Nickel in einem protischen Lösungsmittel wie Methanol oder Ethanol hydriert.

Die bei dieser Hydrierung erhaltene 2-Aminoverbindung wird anschließend mit Glyoxylsäure bei Raumtemperatur und einem pH-Wert von 3—5, der z.B. durch eine wässrige Kaliumcarbonatlösung vorgegeben wird, umgesetzt.

Der hierbei erhaltene $R^A$-substituierte 4-Alkoxymethyl-1.2.3.4-tetrahydro-β-carbolin-1-carbonsäure-3-carbonsäurealkylester der Formel X wird in einem hochsiedenden inerten Lösungsmittel wie Xylol oder Mesitylen in der Siedehitze decarboxyliert und anschließend dehydriert.

Eine Methode zur Dehydrierung besteht darin, daß man das Ausgangsmaterial in einem inerten Lösungsmittel löst bzw. suspendiert und anschließend elementaren Schwefel zugibt, dessen Menge in etwa so bemessen ist, daß pro Doppelbindung ein Moläquivalent Schwefel verwendet wird. Ein geringer Überschuß ist zweckmäßig. Als inerte Lösungsmittel sind an sich alle aprotischen Lösungsmittel geeignet, deren Siedepunkt über 100°C liegt und die gegenüber dem Ausgangsmaterial inert sind. Genannt sei beispielsweise Xylol, Dioxan, Tetrahydrofuran, Methylenchlorid oder Dimethoxyäthan bei Temperaturen zwischen 0 und 60°C mit Reaktionszeiten von 0,5 bis 4 Stunden.

Die Aufarbeitung des Reaktionsgemisches erfolgt bei den jeweiligen Verfahren nach allgemein bekannten Methoden wie Extraktion, Kristallisation, Chromatographie usw.

Die nachfolgenden Beispiele sollen das erfindungsgemäße Verfahren erläutern.

Beispiel 1

1,9 g (5mMol) 6-Jod-4-methyl-β-carbolin-3-carbonsäureethylester werden in 30 ml Dimethylformamid mit 0,87 ml Triethylamin, 22 mg Palladium(II)acetat, 152 mg Tri-ortho-tolylphosphin und 6 ml Cyclohexen versetzt und in einem Druckgefäß unter Argon 6 Stunden bei 140°C Badtemperatur gerührt. Nach Abdestillieren des Lösungsmittels wird in Essigester/gesättigter Natriumhydrogencarbonatlösung verteilt und vom unlöslichen Anteil abgesaugt. Die Essigesterphase wird mit Wasser gewaschen, getrocknet, filtriert und eingeengt. Der Rückstand wird über Kierselgel mit Toluol:Eisessig:Wasser = 10:10:1 als Laufmittel chromatographiert. Die entsprechenden Fraktionen werden eingeengt, in Methylenchlorid aufgenommen, je einmal mit Natriumhydrogencarbonatlösung und mit gesättigter Kochsalzlösung gewaschen, getrocknet, filtriert und eingeengt. Man erhält 500 mg (38% d. Th.) 6-(1-Cyclohexen-4-yl)-4-methyl-β-carbolin-3-carbonsäureethylester vom Schmelzpunkt 200—201°C.

In analoger Weise werden hergestelt:

4-Methyl-6-(1-cycloocten-1-yl)-β-carbolin-3-carbonsäureethylester vom Schmelzpunkt 196—199°C;

ein Gemisch von 4-Methyl-6-(3- und 4-cyclohepten-1-yl)-β-carbolin-3-carbonsäureethylester vom Schmelzpunkt 193°C;

4-Methyl-6-(3-methyl-1.3-butadienyl)-β-carbolin-3-carbonsäureethylester vom Schmelzpunkt 198—200°C;

4-Methyl-6-(2.3-dimethyl-1.3-butadienyl)-β-carbolin-3-carbonsäureethylester;

ein Gemisch von 5-(1- und 2-Cyclohexen-4-yl)-β-carbolin-3-carbonsäureethylester vom Schmelzpunkt 238—243°C;
ein Gemisch von 4-Ethyl-6-(1- und 2-Cyclohexen-4-yl)-β-carbolin-3-carbonsäureethylester;
4-Ethyl-6-(1-cycloocten-1-yl)-β-carbolin-3-carbonsäureethylester;
ein Gemisch von 6-(1- und 2-Cyclohexen-4-yl)-β-carbolin-3-carbonsäureethylester;
6-(1-Cycloocten-1-yl)-β-carbolin-3-carbonsäureethylester;
ein Gemisch von 4-Methoxymethyl-6-(1- und 2-cyclohexen-4-yl)-β-carbolin-3-carbonsäureethylester vom Schmelzpunkt 152—156°C;
4-Methoxymethyl-6-(3-methyl-1.3-butadienyl)-β-carbolin-3-carbonsäureethylester;
4-Methyl-6-(1-cyclohexen-4-yl)-β-carbolin-3-carbonsäureethylester;
4-Methyl-6-(1-cyclohexen-4-yl)-β-carbolin-3-carbonsäurepropylester;
ein Gemisch von 4-Methyl-6-(1-propyl-1- und 2-penten-1-yl)-β-carbolin-3-carbonsäureethylester vom Schmelzpunkt 142—144°C und
6-(Cyclohexylvinyl)-4-methyl-β-carbolin-3-carbonsäureethylester vom Schmelzpunkt 186—205°C.

Beispiel 2

440 mg (1,31 mMol) 6-(1-Cyclohexen-4-yl)-4-methyl-β-carbolin-3-carbonsäureethylester werden in 20 ml Ethanol mit 3,25 ml 1-n-Kalilauge 1 Stunde am Rückfluß erhitzt. Nach Neutralisation mit Essigsäure und Zugabe von 10 ml Wasser wird der Niederschlag abgesaugt, mit Wasser gewaschen und im Vakuum über Kaliumhydroxid getrocknet. Man erhält 378 mg (94% d. Th.) 6-(1-Cyclohexen-4-yl)-4-methyl-β-carbolin-3-carbonsäure vom Schmelzpunkt 284—285°C.
In analoger Weise werden hergestellt:
6-Cyclohexyl-4-methyl-β-carbolin-3-carbonsäure;
6-(1-Cycloocten-1-yl)-4-methyl-β-carbolin-3-carbonsäure;
ein Gemisch von 6-(1- und 2-Cyclohexen-4-yl)-methoxymethyl-β-carbolin-3-carbonsäure vom Schmelzpunkt 240—244°C und

Beispiel 3

1,36 g (20 mMol) Imidazol werden in 15 ml absolutem Tetrahydrofuran mit 0,36 ml Thionylchlorid in 5 ml absolutem Tetrahydrofuran versetzt. Nach 15 Minuten Rühren bei Raumtemperatur wird vom Niederschlag abgesaugt. Das Filtrat wird zu einer Lösung von 0,33 g (1,08 mMol) 6-(1-Cyclohexen-4-yl)-4-methyl-β-carbolin-3-carbonsäure in 15 ml absolutem Dimethylformamid gegeben. Nach 1 Stunde Rühren bei Raumtemperatur versetzt man mit 1,15 g (13 mMol) Propionamidoxim, destilliert das Tetrahydrofuran ab und erhitzt die Reaktionslösung 3 Stunden am Rückfluß. Nach Abdestillieren des Lösungsmittels verteilt man in Methylenchlorid/gesättigter Natriumhydrogencarbonatlösung, wäscht die organische Phase mit gesättigter Natriumchloridlösung neutral, trocknet über Magnesiumsulfat, filtriert und engt zur Trockne ein. Aus dem Rückstand erhält man nach Säulenchromatographie über Kieselgel mit Methylenchlorid:Ethanol = 10:1 als Laufmittel und Umkristallisation aus Ethanol/Hexan 194 mg (50% d. Th.) 6-(1-Cyclohexen-4-yl)-4-methyl-3-(3-ethyl-1.2.4-oxadiazol-5-yl)-β-carbolin vom Schmelzpunkt 247—248°C.
In analoger Weise werden hergestellt:
6-Cyclohexyl-4-methyl-3-(3-ethyl-1.2.4-oxadiazol-5-yl)-β-carbolin;
ein Gemisch von 6-(1- und 2-Cyclohexen-4-yl)-4-methyl-3-(3-ethyl-1.2.4-oxadiazol-5-yl)-β-carbolin;
6-(1-Cycloocten-1-yl)-4-methyl-3-(3-ethyl-1.2.4-oxadiazol-5-yl)-β-carbolin;
6-(3-Methylbut-1-yl)-4-methyl-4-methyl-3-(3-ethyl-1.2.4-oxadiazol-5-yl)-β-carbolin;
ein Gemisch von 6-(1- und 2-Cyclohexen-4-yl)-4-methoxymethyl-3-(3-Ethyl-1.2.4-oxadiazol-5-yl)-β-carbolin vom Schmelzpunkt 142—145°C und
6-t-Butyl-4-methyl-3-(3-ethyl-1.2.4-oxadiazol-5-yl)-β-carbolin.

Beispiel 4

100 mg (0,3 mMol) 6-(1-Cyclohexen-4-yl)-4-methyl-β-carbolin-3-carbonsäureethylester werden in 20 ml Ethanol mit 0,1 g Raney-Nickel bei Raumtemperatur und 10 bar 2 Stunden hydriert. Nach Abtrennen vom Katalysator wird eingedampft und aus wenig Ethanol/Hexan umgefällt. Man erhält 55 mg (54,7% d.Th.) 6-Cyclohexyl-4-methyl-β-carbolin-3-carbonsäureethylester vom Schmelzpunkt 191—193°C.
In analoger Weise werden hergestellt:
6-Cycloheptyl-4-methyl-β-carbolin-3-carbonsäureethylester;
6-Cyclooctyl-4-methyl-β-carbolin-3-carbonsäureethylester;
6-Cyclohexyl-4-methoxymethyl-β-carbolin-3-carbonsäureethylester;
5-Cyclohexyl-β-carbolin-3-carbonsäureethylester vom Schmelzpunkt 254—266°C.

Beispiel 5

435 mg (1,3 mMol) 6-(1-Cyclohexen-4-yl)-4-methyl-β-carbolin-3-carbonsäureethylester werden in 10 ml Dimethylsulfoxid mit 125 mg Schwefel unter Stickstoff 2 Stunden auf 180°C erhitzt. Nach Eindampfen im Vakuum wird in Methylenchlorid/gesättigter Natriumhydrogencarbonatlösung verteilt. Die Methylenchloridphase wird eingedampft und der Rückstand über Kieselgel mit

Toluol:Eisessig:Wasser = 10:10:1 als Laufmittel chromatographiert. Nach Endampfen der entsprechenden Fraktionen und Verrühren mit Hexan erhält man 48 mg (8,4% d. Th.) 6-Phenyl-4-methyl-β-carbolin-3-carbonsäureethylester vom Schmelzpunkt 241—242°C.

Beispiel 6

250 m 4-Phenylindol in 1 ml Eisessig werden bei Raumtemperatur zu einer bei 0°C hergestellten Lösung von 1,1 g 3-Dimethylamino-2-(dimethylaminomethylenamino)-acrylsäureethylester in 10 ml Eisessig gegeben und 20 Stunden auf 100°C erhitzt. Nach Eindampfen und mehrfache Chromatographie über Kieselgel nacheinander mit Methylenchlorid:Ethanol = 95.5, Toluol:Eisssig:Wasser = 10:10:1 und Methylenchlorid:Ethanol = 10:2 erhält man 21,4 mg 5-Phenyl-β-carbolin-3-carbonsäureethylester.

Beispiel 7

260 mg 6-Benzoyl-4-methyl-4-methyl-β-carbolin-3-carbonsäureethylester werden in 36 ml absolutem Ethanol mit 2 ml Eisessig und 100 mg Palladium-Mohr 2 Stunden mit 10 bar Wasserstoffdruck bei 40—45°C hydriert. nach Abtrennen dez Katalysators wird eingedampft. Den Rückstand chromatographiert man über Kieselgel zunächst mit Toluol:Eisessig:Wasser = 10:10:1 und anschließend mit Toluol:Ethanol:Wasser = 80:20:1. Man erhält 27 mg (11%) 6-Benzyl-4-methyl-β-carbolin-3-carbonsäureethylester.

Beispiel 8

2,80 g (13,5 mMol) 4-Benzylindol werden mit 7,48 g (18 mMol) 50%iger Lösung von 4-Methoxy-3-hydroxy-2-nitro-buttersäureethylester in 84 ml Toluol mit 8,3 ml Eisessig unter Argon 15 Stunden gekocht. Nach Abkühlen wird mit Essigester verdünnt und mit Wasser gewaschen.

Die organische Phase wird getrocknet, filtriert und eingeengt und der Rückstand über Kieselgel mit Methylenchlorid als Laufmittel chromatographiert. Man erhält 4,7 g (87%) 3-(4-Benzylindol-3-yl)-2-nitro-5-oxa-hexansäureethylester, der in 50 ml Ethanol mit 4,7 g Raney-Nickel B 115—Z bei Normaldruck und Raumtemperatur hydriert wird. Nach Abtrennen des Katalysators und Endampfen wird über Kieselgel mit Methylenchlorid:Ethanol = 10:1 chromatographiert. Man erhält 1,16 g (24%) 2-Amino-3-(4-benzylindol-3-yl)-5-oxahexansäureethylester, der mit 349 mg Glyoxylsäurehydrat in 4 ml Essigester und 4 ml Wasser versetzt wird, mit 10%iger Kaliumcarbonatlösung auf pH 4 gestellt und über Nacht bei Raumtemperatur gerührt. Nach Abdestillieren des Essigesters wird mit Wasser verdünnt, mit Methylenchlorid extrahiert und die organische Phase getrocknet, filtriert und eingeengt. Man erhält 1,7 g Rohprodukt, das ohne weitere Reinigung in 40 ml Xylol 5 Stunden am Rückfluß gekocht wird. Nach Einengen wird in 40 ml Dimethylsulfoxid aufgenommen, mit 200 mg Schwefel versetzt und 75 Minuten auf 140°C erhitzt. Nach Abdestillieren des Dimethylsulfoxids wird über Kieselgel zweimal zunächst mit Methylenchlorid:Ethanol = 10:1 und dann nochmals mit Hexan:Aceton = 1:1 chromatographiert. Man erhält 45 mg 5-Benzyl-4-methoxymethyl-β-carbolin-3-carbonsäureethylester vom Schmelzpunkt 160—168°C.

In gleicher Weise wird hergestelt:

5-Phenyl-4-methyl-β-carbolin-3-carbonsäureethylester.

Beispiel 9

1,08 g (3 mMol) 6-Jod-4-methyl-β-carbolin-3-carbonsäureethylester werden mit 72 mg (0,06 mMol) Palladium(II)acetat, 0,48 ml (3,6 mMol) Triethylamin und 432 mg (4,5 mMol) 2-Cyclohexen-1-on in 10 ml absolutem Dimethylformamid in einem Druckgefäß 3 Stunden auf 140°C erhitzt. Nach Einengen wird über Kieselgel mit Methylenchlorid:Ethanol = 10:2 chromatographiert. Man erhält nach Umkristallisation der vereinigten polaren Fraktionen aus Methylenchlorid/Cyclohexan 75 mg (6,7% d.Th.) 6-(1-Oxo-2-cyclohexen-3-yl)-4-methyl-β-carbolin-3-carbonsäureethylester vom Schmelzpunkt 245—248°C. Aus den vereinigten unpolaren Fraktionen, die zweimal über Kieselgel mit Toluol:Eisessig-Wasser = 10:10:1 chromatographiert werden, erhält man nach Umkristallisation aus Essigester 60 mg (5,8% d.Th.) 6-(1-Oxocyclohex-3-yl)-4-methyl-β-carbolin-3-carbonsäureethylester vom Schmelzpunkt 182—189°C.

**Patentansprüche**

1. Substituierte β-Carbolinderivate der allgemeinen Formel I

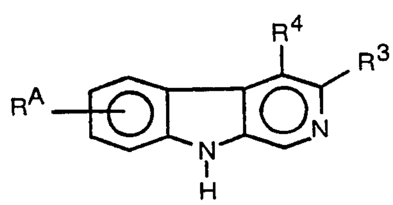

(I),

wobei

$R^3$ einen Oxadiazolylrest der Formel

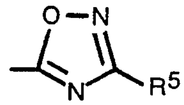

mit $R^5$ als niederem Alkyl mit bis zu 3 C-Atomen oder einen Ester

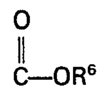

mit $R^6$ als Wasserstoff oder niederem Alkyl mit bis zu 3 C-Atomen,

$R^4$ Wasserstoff, niederes Alkyl mit bis zu 3-C-Atomen oder $CH_2OR^9$ mit $R^9$ als niederem Alkyl mit bis zu 3 C-Atomen,

$R^A$ Phenyl, einen verzweigten oder unverzweigten Alkylrest mit 2—10 Kohlenstoffatomen, einen Phenethyl- oder Cyclohexylvinylrest oder einen Cycloalkyl-, verzweigten oder unverzweigten Alkenyl- oder Cycloalkenylrest mit 2—10 Kohlenstoffatomen, wobei im Cycloalkylrest eine $CH_2$-Gruppe durch Carbonyl oder Sauerstoff ersetzt sein kann, darstellen, wobei, falls $R^A$ einen Phenethylrest oder einen $C_{2-10}$-Alkylrest bedeutet, $R^3$ eine Oxadiazolylrest ist.

2. 6-Cyclohexyl-4-methyl-β-carbolin-3-carbonsäureethylester.

3. 6-(1-Cycloocten-1-yl)-4-methyl-β-carbolin-3-carbonsäureethylester.

4. 6-(1- und 2-Cyclohexen-4-yl)-4-methoxy-methyl-β-carbolin-3-carbonsäureethylester.

5. 6-Benzyl-4-methyl-β-carbolin-3-carbonsäureethylester.

6. 5-Benzyl-4-methyoxymethyl-β-carbolin-3-carbonsäureethylester.

7. 6-(1-Oxocyclohex-3-yl)-methyl-β-carbolin-3-carbonsäureethylester.

8. Verfahren zur Herstellung von substituierten β-Carbolinderivaten der allgemeinen Formel I

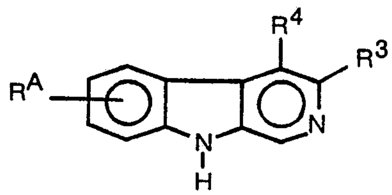

(I),

wobei

$R^3$ einen Oxadiazolylrest der Formel

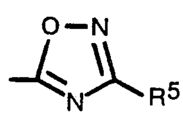

mit $R^5$ als niederem Alkyl mit bis zu 3 C-Atomen oder einen Ester

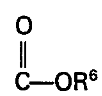

mit $R^6$ als Wasserstoff oder niederem Alkyl mit bis zu 3 C-Atomen,

$R^4$ Wasserstoff, niederes Alkyl mit bis zu 3-C-Atomen oder $CH_2OR^9$ mit $R^9$ als niederem Alkyl mit bis zu 3 C-Atomen,

$R^A$ Phenyl, einen verzweigten oder unverzweigten Alkylrest mit 2—10 Kohlenstoffatomen, einen Phenethyl- oder Cyclohexylvinylrest oder einen Cycloalkyl-, verzweigten oder unverzweigten Alkenyl- oder Cycloalkenylrest mit 2—10 Kohlenstoffatomen, wobei im Cycloalkylrest eine $CH_2$-Gruppe durch Carbonyl oder Sauerstoff ersetzt sein kann, darstellen, wobei, falls $R^A$ einen Phenethylrest oder einen $C_{2-10}$-Alkylrest bedeutet, $R^3$ ein Oxadiazolylrest ist, dadurch gekennzeichnet, daß man

A. ein halogeniertes β-Carbolinderivat der allgemeinen Formel II

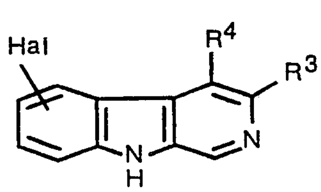

(II),

worin Hal für Brom oder Jod steht und $R^3$ und $R^4$ die oben angegebene Bedeutung haben, mit einem Alkenyl- oder Cycloalkenylrest mit 2—10 C-Atomen, in einem aprotischen polaren Lösungsmittel in Gegenwart eines Schwermetallsalzes und einer Base unter Druck umsetzt, und anschließend gegebenenfalls die im Substituenten $R^A$ vorhandene isolierte oder konjugierte Doppelbindung in Gegenwart von Raney-Nickel oder einem Edelmetallkatalysator auf einem Trägermaterial in einem protischen Lösungsmittel hydriert oder anschließend mit elementarem Schwefel in Dimethylsulfoxid oder mit Palladium in Xylol und/oder Mesitylen dehydriert und anschließend gegebenenfalls eine Estergruppe in 3-Stellung alkalisch hydrolysiert und gegebenenfalls anschließend die so erhaltene freie säure der Formel III

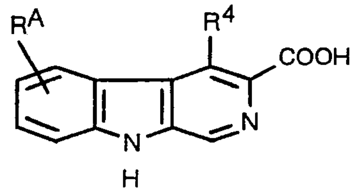

(III),

worin $R^4$ und $R^A$ die oben angegebene Bedeutung haben, mit einem Amidoxim der allgemeinen Formel $R^5$—C(=NOH)$NH_2$, worin $R^5$ die oben angegebene Bedeutung hat, in einem inerten Lösungsmittel bei Raumtemperatur bis zur Siedetemperatur des Reaktionsgemisches umsetzt, oder

B. ein β-Carbolinderivat der Formel IV

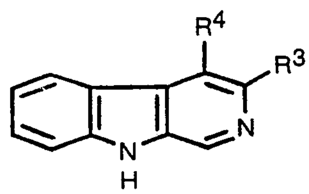

(IV),

wobei $R^3$ und $R^4$ die oben angegebene Bedeutung haben, mit einem Alkylhalogenid mit 2 bis 10 C-Atomen und Halogenid in der Bedeutung von Chlor oder Brom in Gegenwart von Aluminiumtrichlorid bei Raumtemperatur alkyliert oder

C. ein substituiertes β-Carbolinderivat der Formel V

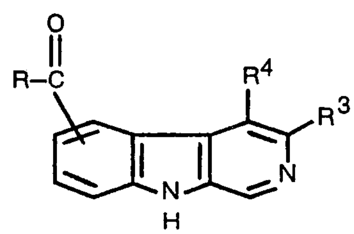

(V),

wobei R Wasserstoff oder einen Kohlenwasserstoffrest mit bis zu 9 C-Atomen darstellt und $R^3$ und $R^4$ die oben angegebene Bedeutung haben, in Gegenwart von Palladium in feinverteilter Form in einem aliphatischen Alkohol und Eisessig bei Temperaturen zwischen 20 und 100°C und Drücken von 5—20 bar hydriert oder

D. ein substituiertes Indol der Formel VI

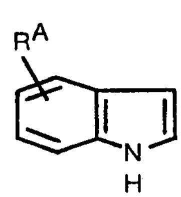

(VI),

worin $R^A$ die oben angegebene Bedeutung hat, mit einem Azadien der Formel VII

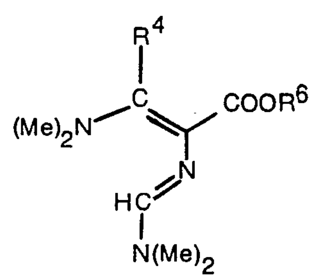

(VII),

wobei $R^4$ die oben angegebene Bedeutung hat und $R^6$ einen niederen Alkylrest mit bis zu 3 C-Atomen darstellt, in Gegenwart einer Säure bei Temperaturen von 50—200°C umsetzt oder

E. ein substituiertes Indol der Formel VI mit einem 4-Alkoxy-3-hydroxy-2-nitro-buttersäurealkylester der Formel VIII

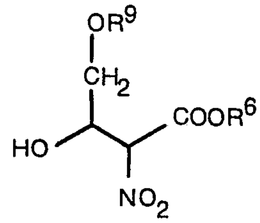

(VIII),

worin $R^6$ und $R^9$ die oben angegebene Bedeutung haben, in einem inerten Lösungsmittel bei Rückflußtemperatur in Gegenwart einer aliphatischen Carbonsäure erhitzt, anschießend den so erhaltenen 3-Indol-3-yl)-2-nitro-5-oxa-hexanalkylester der Formel IX

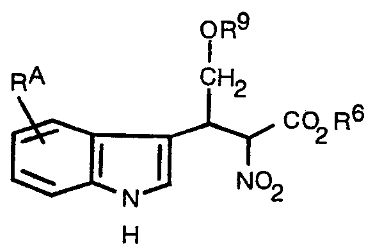

(IX),

worin $R^6$ und $R^9$ die oben angegebene Bedeutung haben, in Gegenwart von Raney-Nickel bei Raumtemperatur und unter Normaldruck zur entsprechenden 2-Amin -Verbindung hydriert, mit Glyoxylsäure bei einem pH von 3—5 bei Raumtemperatur umsetzt, wobei ein $R^A$-substituierter 4-Alkoxymethyl-1.2.3.4-tetrahydro-β-carbolin-1-carbonsäure-3-carbonsäurealkylester der Formel X

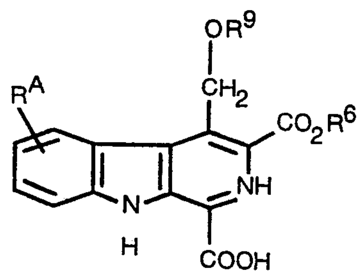

(X),

worin $R^6$ und $R^9$ und $R^A$ die oben angegebene Bedeutung haben, entsteht, der anschließend in einem hochsiedenden inerten Lösungsmittel durch Erhitzen auf Rückflußtemperatur decarboxyliert und anschließend dehydriert.

9. Arzneimittel auf Basis von Verbindungen gemäß den Ansprüchen 1—7.

**Revendications**

1. β-Carbolines substituées répondant à la formule générale I:

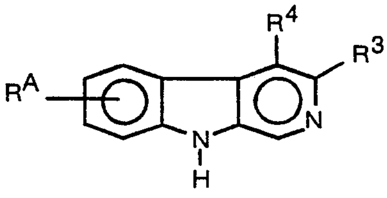

(I),

dans laquelle:

$R^3$ représente un radical oxadizolyle de formule:

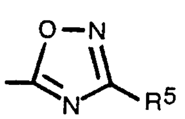

(dans lequel $R^5$ désigne un alkyle inférieure contenant au maximum 3 atomes de carbone) ou un radical d'ester

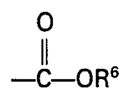

(dans lequel $R^6$ désigne l'hydrogène ou un alkyle inférieur contenant au maximum 3 aomes de carbone),

$R^4$ représente l'hydrogène, unalkyle inférieur contenant au maximum 3 atomes de carbone ou un radical $—CH_2OR^9$ (dans lequel $R^9$ désigne un alkyle inférieur contenant au plus 3 atomes de carbone), et

$R^A$ représente un radical phényle, un radical alkyle ramifié ou non qui contient de 2 à 10 atomes de carbone, un radical phénéthyle ou cyclohexylvinyle, ou un radical cycloalkyle, alcényle ramifié ou non ou cycloalcényle contenant de 2 à 10 atomes de carbone, un radical $—CH_2—$ du radical cycloalkyle pouvant être remplacé par un carbonyle ou par l'oxygène, avec la condition que $R^3$ représente un radical oxadiazolyle dans le cas où $R^A$ représente un radical phénéthyle ou un radical alkyle en $C_2—C_{10}$.

2. Cyclohexyl-6 méthyl-4 β-carboline-carboxylate-3 d'éthyle.

3. (Cyclo-octène-1 yl)-6 méthyl-4 β-carboline-carboxylate-3 d'éthyle.

4. (Cyclohexène-3 et -2)-6 méthoxyméthyl-4 β-carboline-carboxylates-3 d'éthyle.

5. Benzyl-6 méthyl-4 β-carboline-carboxylate-3 d'éthyle.

6. Benzyl-5 méthoxyméthyl-4 β-carboline-carboxylate-3 d'éthyle.

7. (Oxo-3 cyclohexyl)-6 méthyl-4 β-carboline-carboxylate-3 d'éthyle.

8. Procédé pour préparer des β-carbolines substituées répondant à la formule générale I:

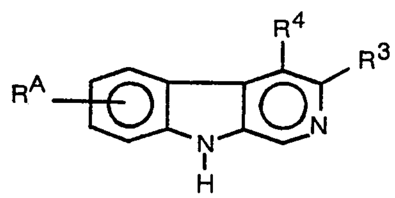

(I),

dans laquelle:

$R^3$ représente un radical oxadiazolyle de formule:

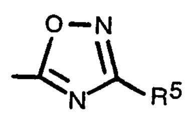

(dans lequel $R^5$ désigne un alkyle inférieur contenant au maximu 3 atomes de carbone) ou un radical d'ester

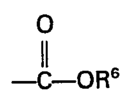

(dans lequel $R^6$ désigne l'hydrogène ou un alkyle inférieur contenant au maximum 3 atomes de carbone),

$R^4$ représente l'hydrogène, un alkyle inférieur contenant au maximum 3 atomes de carbone ou un radical $—CH_2OR^9$ (dans lequel $R^9$ désigne un alkyle inférieur contenant au plus 3 atomes de carbone), et

$R^A$ représente un radical phényle, un radical alkyle ramifié ou non qui contient de 2 à 10 atomes de carbone, un radical phénéthyle ou cyclohexylvinyle, ou un radical cycloalkyle, alcényle ramifié ou non ou cycloalcényle contenant de 2 à 10 atomes de carbone, un radical $—CH_2—$ du radical cycloalkyle pouvant être emplacé par un carbonyle ou pa l'oxygène, avec la condition que $R^3$ représente un radical oxadiazolyle dans le cas où $R^A$ représente un radical phénéthyle ou un radical alkyle en $C_2—C_{10}$, procédé caractérisé en ce que:

A. On fait réagir sous pression une β-carboline halogénée répondant à la formule générale II:

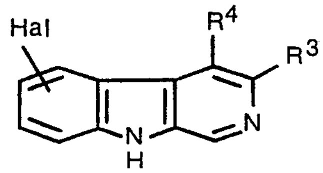

(II),

dans laquelle Hal représente le brome ou l'iode, et $R^3$ et $R^4$ ont les significations qui leur ont été données ci-dessus,

avec un radical alcényle ou cycloalcényle contenant de 2 à 10 atomes de carbone, dans un solvant polaire aprotique, en présence d'un sel de métal lourd et d'une base, et ensuite, éventuellement on hydrogène la

double liaison isolée ou conjuguée qui se trouve dans le substituant $R^A$, en présence de nickel de Raney ou d'un catalyseur constitué d'un métal noble déposé sur un support, dans un solvant protique, ou on déshydrogène ensuite à l'aide de soufre élémentaire dans du diméthylsulfoxyde ou au moyen de palladium dans du xylène et/ou du mésitylène, et on hydrolyse ensuite éventuellement, en milieu alcalin, un radical d'ester en position 3, après quoi, éventuellement, on fait réagir l'acide libre ainsi obtenu, qui répond à la formule III:

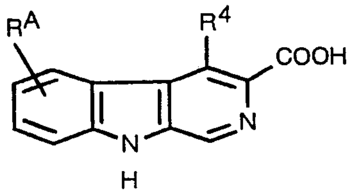

(III),

dans laquelle $R^4$ et $R^A$ ont les significations précédemment données,
avec une amide-oxime répondant à la formule générale:

$$R^5—C(=NOH)NH_2$$

dans laquelle $R^5$ a la signification qui lui a été donnée ci-dessus,
dans un solvant inerte, à une température comprise entre la température ambiante et la température d'ébullition du mélange réactionnel, ou

B. On alkyle à la température ambiante une β-carboline répondant à la formule IV:

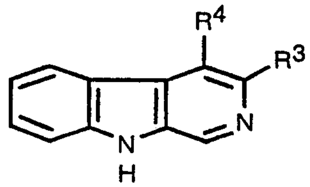

(IV),

dans laquelle $R^3$ et $R^4$ ont les significations qui leur ont été données ci-dessus,
avec un halogénure d'alkyle dont l'halogène est e chlore ou le brome et dont l'alkyle contient de 2 à 10 atomes de carbone ou avec un alcène en $C_2—C_{10}$, en présence de trichlorure d'aluminium, ou

C. On hydrogène une β-carboline substituee répondant à la formule V:

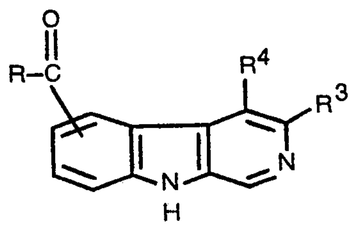

(V),

dans laquelle R représente l'hydrogène ou un radical hydrocarboné contenant au maximum 9 atomes de carbone, et $R^3$ et $R^4$ ont les significations qui leur ont été données ci-dessus,
en présence de palladium à l'état finement divisé, dans un alcool aliphatique et l'acide acétique glacial, à des températures comprises entre 20 et 100°C et sous des pressions de 5 à 20 bar, ou

D. On fait réagir un indole substitué répondant à la formule VI:

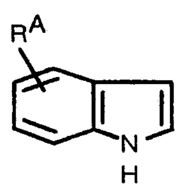

(VI),

dans laquelle $R^A$ a la signification qui lui a été donnée ci-dessus, avec un azadiène répondant à la formule VII:

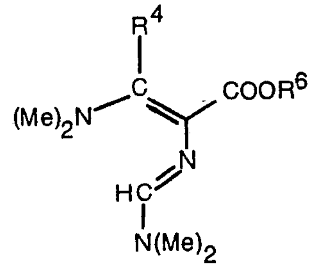

(VII),

dans laquelle $R^4$ a la signification qui lui a été donnée ci-dessus et $R^6$ représente un radical alkyle inférieur contenant au plus 3 atomes de carbone, en présence d'un acide, à des températures de 50 à 200°C, ou

E. On chauffe un indole substitué de formule VI avec un alcoxy-4-hydroxy-3-nitro-2 butyrate d'alkyle répondant àb la formule VIII:

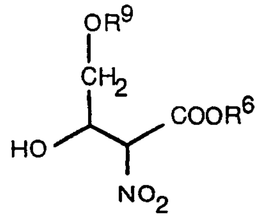

(VIII),

dans laquelle $R^6$ et $R^9$ ont les significations qui leur ont été données ci-dessus, dans un solvant inerte, à la température du reflux, en présence d'un acide carboxylique aliphatique, puis on hydrogène l'(indolyl-3)-3 nitro-2 oxa-5 hexanoate d'alkyle ainsi obtenu, qui répond à la formule IX:

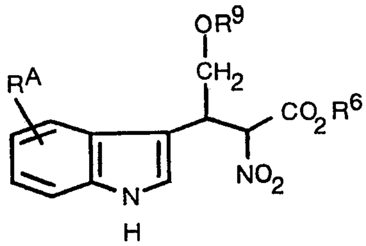

(IX),

dans laquelle $R^6$ et $R^9$ ont les significations indiquées ci-dessus, en présence de nickel de Raney, à la température ambiante et sous la pression normale, de manière à le convertir en le composé aminé en 2 correspondant, on fait réagir avec l'acide glyoxylique à un pH de 3 à 5 à la température ambiante, réaction qui donne naissance à un alcoxyméthyl-4 tétrahydro-1,2,3,4 carboxy-1 β-carboline-carboxylate-3 d'alkyle à substituant $R^A$ qui répond à la formule X:

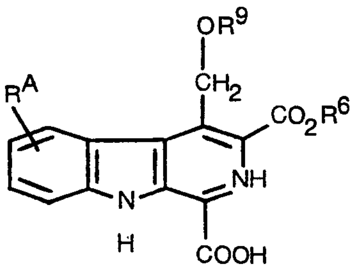

(X),

dans laquelle $R^6$, $R^9$ et $R^A$ ont les significations qui leur ont été données plus haut, après quoi on décarboxyle celui-ci dans un solvant inerte à haut pont d'ébullition par chauffage à la température du reflux et ensuite on le déshydrogène.

9. Médicament à base de composés selon l'une quelconque des revendications 1 à 7.

**Claims**

1. Substituted β-carboline derivatives of general formula I

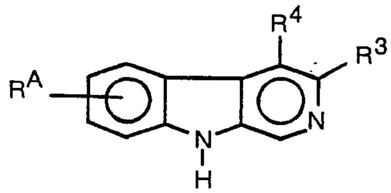

(I),

wherein
$R^3$ is an oxadiazolyl radical of formula

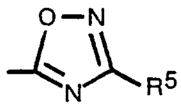

$R^5$ being lower alkyl having up to 3 carbon atoms, or $R^3$ is an ester

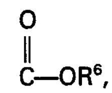

$R^6$ being hydrogen or lower alkyl having up to 3 carbon atoms,
$R^4$ is hydrogen, lower alkyl having up to 3 carbon atoms or $CH_2OR^9$, $R^9$ being lower alkyl having up to 3 carbon atoms,
$R^A$ is phenyl, a branched or unbranched alkyl radical having from 2 to 10 carbon atoms, a phenethyl or cyclohexylvinyl radical or a cycloalkyl, branched or unbranched alkenyl or cycloalkenyl radical having from 2 to 10 carbon atoms, wherein a $CH_2$ group in the cycloalkyl radical may be replaced by carbonyl or oxygen, and wherein, if $R^A$ is a phenethyl radical or a $C_{2-10}$-alkyl radical, $R^3$ is an oxadiazolyl radical.
2. 6-cyclohexyl-4-methyl-β-carboline-3-carboxylic acid ethyl ester.
3. 6-(1-cycloocten-1-yl)-4-methyl-β-carboline-3-carboxylic acid ethyl ester.
4. 6-(1- and 2-cyclohexen-4-yl)-4-methoxymethyl-β-carboline-3-carboxylic acid ethyl ester.
5. 6-benzyl-4-methyl-β-carboline-3-carboxylic acid ethyl ester.
6. 5-benzyl-4-methoxymethyl-β-carboline-3-carboxylic acid ethyl ester.
7. 6-(1-oxocyclohex-3-yl)-4-methyl-β-carboline-3-carboxylic acid ethyl ester.
8. Process for the preparation of substituted β-carboline derivatives of general formula I

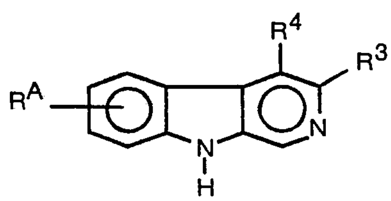

(I),

wherein
$R^3$ is an oxadiazolyl radical of formula

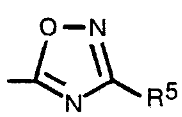

$R^5$ being lower alkyl having up to 3 carbon atoms, or $R^3$ is an ester

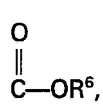

$R^6$ being hydrogen or lower alkyl having up to 3 carbon atoms,
$R^4$ is hydrogen, lower alkyl having up to 3 carbon atoms or $CH_2OR^9$, $R^9$ being lower alkyl having up to 3 carbon atoms,
$R^A$ is phenyl, a branched or unbranched alkyl radical having from 2 to 10 carbon atoms, a phenethyl or cyclohexylvinyl radical or a cycloalkyl, branched or unbranched alkenyl or cycloalkenyl radical having from

2 to 10 carbon atoms, wherein a $CH_2$ group in the cycloalkyl radical may be replaced by carbonyl or oxygen, and wherein, if $R^A$ is a phenethyl radical or a $C_{2-10}$-alkyl radical, $R^3$ is an oxadiazolyl radical, characterised in that

A. a halogenated β-carboline derivative of general formula II

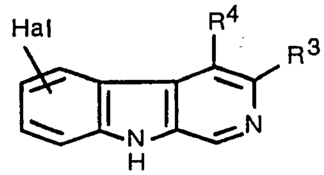

(II),

wherein Hal is bromine or iodine and $R^3$ and $R^4$ are as defined above, is reacted under pressure in an aprotic polar solvent in the presence of a heavy metal salt and a base with an alkenyl or cycloalkenyl radical having from 2 to 10 carbon atoms, and then optionally the isolated or conjugated double bond present in the $R^A$ substituent is hydrogenated in a protonic solvent in the presence of Raney nickel or a noble metal catalyst on a support material, or subsequently dehydrogenated with elemental sulphur in dimethyl sulphoxide or with palladium in xylene and/or mesitylene, and then optionally an ester group in the 3-position is subjected to alkaline hydrolysis, and optionally the resulting free acid of formula III

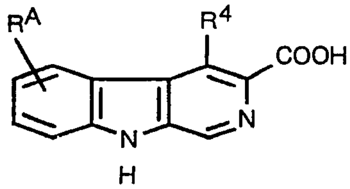

(III),

wherein $R^4$ and $R^A$ are as defined above, is then reacted in an inert solvent at from room temperature to the boiling temperature of the reaction mixture with an amidoxime of general formula $R^5—C(=NOH)NH_2$, wherein $R^5$ is as defined above, or

B. a β-carboline derivative of formula IV

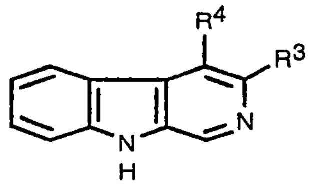

(IV),

wherein $R^3$ and $R^4$ are as defined above, is alkylated at room temperature in the presence of aluminium trichloride with an alkyl halide having from 2 to 10 carbon atoms, the halide being a chloride or bromide, or

C. a substituted β-carboline derivative of formula V

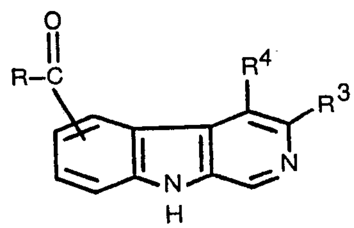

(V),

wherein R is hydrogen or a hydrocarbon radical having up to 9 carbon atoms, and

$R^3$ and $R^4$ are as defined above, is hydrogenated in the presence of palladium in finely divided form in an aliphatic alcohol and glacial acetic acid at temperatures of between 20 and 100°C and pressures of from 5 to 20 bar, or

D. a substituted indole of formula VI

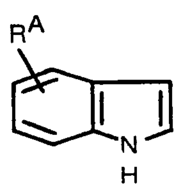

(VI),

wherein $R^A$ is as defiend above, is reacted in the presence of an acid at temperatures of from 50 to 200°C with an azadiene of formula VII

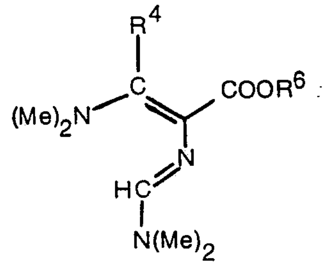

(VII),

wherein $R^4$ is as defined above, and $R^6$ is a lower alkyl radical having up to 3 carbon atoms, or

E. a substituted indole of formula VI is heated in an inert solvent at reflux temperature in the presence of an aliphatic carboxylic acid with a 4-alkoxy-3-hydroxy-2-nitrobutyric acid alkyl ester of formula VIII

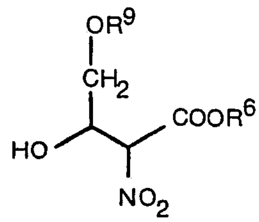

(VIII),

wherein $R^6$ and $R^9$ are as defined above, and then the resulting 3-(indol-3-yl)-2-nitro-5-oxa-hexanoic alkyl ester of formula IX

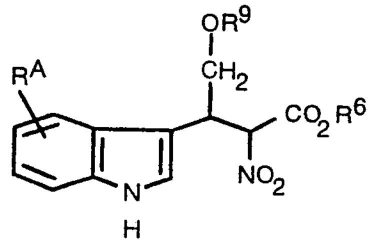

(IX),

wherein $R^6$ and $R^9$ are as defined above, is hydrogenated in the presence of Raney nickel at room temperature and under normal pressure to the corresponding 2-amine compound, and reacted at room temperature with glyoxylic acid at a pH of from 3 to 5, to produce an $R^A$-substituted 4-alkoxymethyl-1,2,3,4-tetrahydro-β-carboline-1-carboxylic acid 3-carboxylic acid alkyl ester of formula X

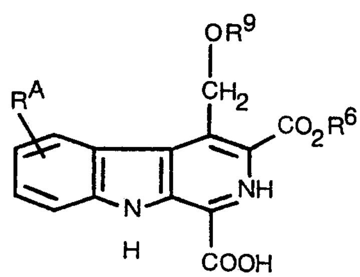

(X),

wherein $R^6$, $R^9$ and $R^A$ are as defined above, and subsequently this ester is decarboxylated in a high-boiling inert solvent by heating to reflux temperature and is then dehydrogenated.

9. Medicament based on compounds according to claims 1 to 7.